# EUROPEAN PATENT APPLICATION

(11) **EP 4 585 208 A1**
(43) Date of publication of application: **16.07.2025**
(21) Application number: 23878814.5
(22) Date of filing: 25.08.2023
(51) Int. Cl.: A61K 9/127, A61K 8/14, A61K 47/36, A61K 8/73

(54) **HYALURONIC ACID LIPOSOME ASSEMBLY, AND PREPARATION METHOD THEREFOR AND USE THEREOF**

(30) Priority: 20.10.2022 CN 202211286379
(71) Applicant: HONEST MEDICAL CHINA CO., LTD., Zhuhai, Guangdong 519031 (CN); Ushare Medical Inc., Zhuhai, Guangdong 519031 (CN)
(72) Inventor: WANG, Jianjin, Zhuhai, Guangdong 519031 (CN); LI, Guowei, Zhuhai, Guangdong 519031 (CN); XING, Hui, Zhuhai, Guangdong 519031 (CN); MA, Dong, Zhuhai, Guangdong 519031 (CN); GUO, Aijun, Zhuhai, Guangdong 519031 (CN); LEI, Chi Weng, Zhuhai, Guangdong 519031 (CN)
(74) Representative: Primiceri, Maria Vittoria
(86) International application number: PCT/CN2023/115010
(87) International publication number: WO 2024/082819

(57) **Abstract**

The present disclosure provides a hyaluronic acid liposome assembly, and a preparation method therefor and a use thereof. The hyaluronic acid liposome assembly contains hyaluronic acid and a liposome in a mass ratio of 1:(20-150); the hyaluronic acid liposome assembly is prepared from the liposome and the hyaluronic acid of different molecular weights in a certain assembling manner, so that hyaluronic acid participates in mutual chimerism of the liposome of a double-molecular structure, thereby forming a novel stable double-molecular-layer skeleton structure, i.e., a hyaluronic acid liposome assembly (HA-liposome, the structure is as shown in figure 1). Under the dual effects of skin moisturizing and permeation promotion of the hyaluronic acid, the hyaluronic acid further cooperates with the liposome, so that the effect of efficient permeation of the HA-liposome on the skin is achieved.

## Description

### TECHNICAL FIELD

The present disclosure relates to the field of pharmaceuticals and daily chemicals, in particular to a hyaluronic acid liposome assembly, a preparation method, and use thereof.

### BACKGROUND

A transdermal drug delivery system (TDDS) is a novel drug formulation, when administered on skin surface, the drug permeates all layers of the skin at a certain rate and enters blood circulation throughout the human body via capillaries to achieve an effective blood concentration, thus achieving a systemic or local therapeutic effect. Compared with oral administration, intravenous injection, or other modes of administration, transdermal drug delivery has the advantages of avoiding a first-pass effect in gastrointestinal tract, reducing a fluctuation of a blood concentration, avoiding a stimulation of the drug to gastrointestinal tract, being non-invasive during administration, well adaptable, efficient, and convenient, etc. However, the skin is a highly impermeable barrier to most drugs, and many drugs have difficulty permeating the skin, or a permeation amount hardly reaches a therapeutic level. Therefore, promoting transdermal permeation of drugs by appropriate methods has become crucial for TDDS. At present, the methods to achieve efficient permeation of drugs are mainly divided into chemical methods by adding natural or chemically synthesized osmotic agents, and physical methods by using physical technologies such as sound, light, forces, electricity, and heat or by using microneedles, subcutaneous needles, etc., to reduce the hindrance of the skin to drug absorption. However, the drug permeation efficiency of chemical methods is easily affected by penetrants, and efficient penetrants often have relatively great irritation and side effects, causing certain irritation and sensitization to the skin. Physical methods have relatively high costs and technical requirements and are highly likely to cause phenomena such as skin pain, mild bleeding, wound suppuration, and the occurrence of a burning sensation on the face. Therefore, safe and efficient novel transdermal formulations have become the main research direction in recent years.

Liposome is prepared by dispersing phospholipid and other amphiphilic substances in water, in which one or more layers of concentric lipid bimolecular membranes encapsulate into closed vesicles. Liposome is widely used in intravenous, cutaneous, pulmonary, and oral administrations, etc., due to its advantages of an excellent drug encapsulation and loading capacity, a good skin friendliness, a good skin permeability, the ability to prolong the drug release, a good biological safety, etc. However, in view of the tight "brick wall" structure of the skin, how to further improve the drug permeability of liposome into the skin and maximize the therapeutic effect of the drugs has become a key issue for liposome transdermal drug delivery formulations.

Hyaluronic acid, also known as hyaluronan, is a glycosaminoglycan of disaccharide units composed of D-glucuronic acid and N-acetylglucosamine. Due to the characteristics of a good biocompatibility, non-immunogenicity, biodegradability, viscoelasticity, etc., hyaluronic acid is widely used in biomedicine such as antimicrobial and anti-inflammatory actions, wound repair and healing, joint lubrication, bone filling, slow release of drugs, and tissue engineering.

Since the human skin contains oxides or hyaluronidase, hyaluronic acids with different molecular weights have great differences in skin permeability and residence time, which leads to significant differences in water-locking and moisturizing properties between the hyaluronic acids with different molecular weights. However, most transdermal technologies involving hyaluronic acid currently on the market suggest that the hyaluronic acid with high molecular weights can only form a protective film on the outer layer of the skin and cannot achieve effective skin permeation. Furthermore, there have been no reports in relevant research. Therefore, how to achieve efficient permeation and long-term residence of the hyaluronic acid with a large molecular weight (1,000,000-3,000,000) in the skin has become crucial to enhance the ability of hyaluronic acid improving the skin performance and reshape the market perception.

### SUMMARY

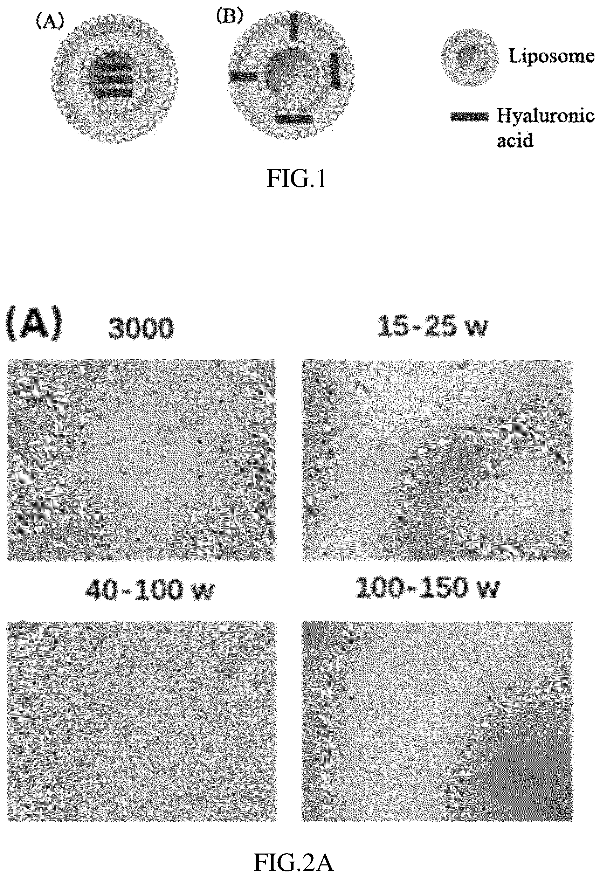

The existing hyaluronic acid liposome assemblies are basically composed of hyaluronic acid wrapped in liposome, forming a structure as shown in Panel A of FIG. 1. In the present disclosure, we have realized that hyaluronic acid directly participates in formation of a liposome scaffold by means of a special process, thereby forming a structure in Panel B of FIG. 1. The new structure is crucial to a transdermal effect of the hyaluronic acid, especially a transdermal effect of the hyaluronic acid with a high molecular weight. Therefore, the present disclosure claims and explores the transdermal administration of hyaluronic acids with different molecular weights, especially the hyaluronic acid with a large molecular weight and the preparation of dosage forms thereof.

An objective of the present disclosure is to provide a hyaluronic acid liposome assembly.

Another objective of the present disclosure is to provide a method for preparing the hyaluronic acid liposome assembly.

Another objective of the present disclosure is to provide use of the hyaluronic acid liposome assembly.

In order to achieve the above objectives, the present disclosure provides, in one aspect, a hyaluronic acid liposome assembly, wherein the hyaluronic acid liposome assembly comprises hyaluronic acid and liposome at a mass ratio of 1:20-150; the hyaluronic acid is located in a bimolecular layer of the liposome and constitutes the bimolecular layer of the liposome with a hyaluronic acid scaffold structure; and a mass of the liposome is based on a mass of phospholipid in the liposome.

The hyaluronic acid liposome assembly according to the present disclosure is assembled by liposome and hyaluronic acid in a certain manner, such that the hyaluronic acid participates in a construction of the liposome with a bimolecular layer structure during which the hyaluronic acid and the liposome jointly form the bimolecular layer structure, and further forms a novel and stable bimolecular layer scaffold structure, namely a hyaluronic acid liposome assembly (HA-Liposome assembly, with a structure as shown in FIG. 1).

In the present disclosure, a series of hyaluronic acid liposome assemblies (HA-liposome assemblies) with hyaluronic acids having different molecular weights are constructed. By adjusting a ratio of components in the hyaluronic acid liposome assembly and conditions for assembling, a series of hyaluronic acid liposome assemblies with hyaluronic acids having different molecular weights have been prepared. A transdermal experiment study has been carried out in vitro to explore the transdermal performance of the hyaluronic acid liposome assemblies and assess the change of the transdermal performance of the hyaluronic acid liposome assemblies on hyaluronic acids having different molecular weights, which provides a new direction for comprehensively studying the permeability of a hyaluronic acid transdermal drug delivery formulation to further obtain a novel material that can achieve efficient skin permeation.

Unlike the published hyaluronic acid liposome composite system, a class of hyaluronic acid liposome assemblies with an innovative structure are constructed in the present disclosure, in which hyaluronic acid participates in a formation of the liposome itself as a scaffold molecule instead of being wrapped in a central cavity of the liposome.

It can be understood that the phrase "a mass of the liposome is based on a mass of phospholipid in the liposome" as described in the present disclosure means that a mass ratio of the hyaluronic acid to the phospholipid in the liposome is 1:20-150.

According to some specific embodiments of the present disclosure, the hyaluronic acid liposome assembly comprises hyaluronic acid and liposome at a mass ratio of 1:40-100.

According to some specific embodiments of the present disclosure, the hyaluronic acid has a molecular weight of 3,000-1,500,000.

According to some specific embodiments of the present disclosure, the hyaluronic acid is at least one of or a combination of some of hyaluronic acids having the following molecular weight ranges: a hyaluronic acid with a molecular weight of 3000, a hyaluronic acid with a molecular weight of 150,000-250,000, a hyaluronic acid with a molecular weight of 400,000-1,000,000, and a hyaluronic acid with a molecular weight of 1,000,000-1,500,000.

According to some specific embodiments of the present disclosure, the liposome is composed of cholesterol and phospholipid at a mass ratio of 1:5-30.

According to some specific embodiments of the present disclosure, the phospholipid is one or more of soybean lecithin, dioleoyl lecithin, and egg yolk lecithin.

According to some specific embodiments of the present disclosure, the liposome is composed of cholesterol and phospholipid at a mass ratio of 1: 10-25.

In another aspect, the present disclosure further provides a method for preparing the hyaluronic acid liposome assembly, comprising the following steps:
preparing a liposome solution;
preparing a hyaluronic acid solution; and
adding the hyaluronic acid solution to the liposome solution under stirring condition to form the hyaluronic acid liposome assembly.

According to some specific embodiments of the present disclosure, the step of preparing the liposome solution comprises dissolving liposome in a solvent and stabilizing a resulting solution to obtain the liposome solution.

According to some specific embodiments of the present disclosure, the step of preparing the liposome solution comprises dissolving liposome in a solvent at 23-32 °C and stabilizing a resulting solution to obtain the liposome solution.

According to some specific embodiments of the present disclosure, the step of preparing the liposome solution comprises dissolving liposome in a solvent at 25-30 °C and stabilizing a resulting solution to obtain the liposome solution.

According to some specific embodiments of the present disclosure, a solvent used for dissolving the liposome is ethanol, diethyl ether, or chloroform.

According to some specific embodiments of the present disclosure, on the basis of the phospholipid in the liposome, an amount of the solvent used for dissolving the liposome is that every 10 mL of the solvent dissolves 0.1-0.8 g of the phospholipid.

According to some specific embodiments of the present disclosure, on the basis of the phospholipid in the liposome, an amount of the solvent used for dissolving the liposome is that every 10 mL of the solvent dissolves 0.1-0.5 g of the phospholipid.

According to some specific embodiments of the present disclosure, on the basis of the phospholipid in the liposome, an amount of the solvent used for dissolving the liposome is that every 10 mL of the solvent dissolves 0.1-0.4 g of the phospholipid.

According to some specific embodiments of the present disclosure, the liposome solution is obtained by stabilization for 1.5-10 h after the liposome is dissolved in the solvent.

According to some specific embodiments of the present disclosure, the liposome solution is obtained by stabilization for 2-6 h after the liposome is dissolved in the solvent.

According to some specific embodiments of the present disclosure, the liposome solution is obtained by stabilization for 2-5 h after the liposome is dissolved in the solvent.

According to some specific embodiments of the present disclosure, the step of preparing the hyaluronic acid solution comprises dissolving hyaluronic acid in a buffer solution or water until uniformly dissolved to obtain the hyaluronic acid solution.

According to some specific embodiments of the present disclosure, the step of preparing the hyaluronic acid solution comprises dissolving hyaluronic acid in a buffer solution at 23-32 °C and stabilizing a resulting solution for uniform dissolution to obtain the hyaluronic acid solution.

According to some specific embodiments of the present disclosure, the step of preparing the hyaluronic acid solution comprises dissolving hyaluronic acid in a buffer solution at 25-30 °C and stabilizing a resulting solution for uniform dissolution to obtain the hyaluronic acid solution.

According to some specific embodiments of the present disclosure, the step of preparing the hyaluronic acid solution comprises dissolving hyaluronic acid in a buffer solution or water, and treating a resulting solution with a shaker or homogenizer to uniformly dissolve the hyaluronic acid to obtain the hyaluronic acid solution.

According to some specific embodiments of the present disclosure, the shaker is operated at a temperature of 20-30 °C for 5-15 min.

According to some specific embodiments of the present disclosure, the buffer solution is a phosphate buffer solution with a concentration of 1-10 mg/mL.

According to some specific embodiments of the present disclosure, on the basis of the hyaluronic acid, an amount of the buffer solution is that every 10 mL of the buffer solution dissolves 10-100 mg of the hyaluronic acid.

According to some specific embodiments of the present disclosure, on the basis of the hyaluronic acid, an amount of the buffer solution is that every 10 mL of the buffer solution dissolves 13-60 mg of the hyaluronic acid.

According to some specific embodiments of the present disclosure, on the basis of the hyaluronic acid, an amount of the buffer solution is that every 10 mL of the buffer solution dissolves 15-50 mg of the hyaluronic acid.

According to some specific embodiments of the present disclosure, the hyaluronic acid is dissolved in the buffer solution until uniformly dissolved and then stabilized for 2-10 h to obtain the hyaluronic acid solution.

According to some specific embodiments of the present disclosure, in the step of preparing the hyaluronic acid solution, the stabilization is carried out for 3-7 h.

According to some specific embodiments of the present disclosure, in the step of preparing the hyaluronic acid solution, the stabilization is carried out for 3-6 h.

According to some specific embodiments of the present disclosure, the hyaluronic acid solution is added to the liposome solution under stirring condition, and the stirring is carried out by a magnetic heating stirrer.

According to some specific embodiments of the present disclosure, in the step of adding the hyaluronic acid solution to the liposome solution under stirring condition to form the hyaluronic acid liposome assembly, the stirring condition includes a stirring speed of 1000-5000 rpm.

According to some specific embodiments of the present disclosure, the above stirring speed is 1000-2000 rpm.

According to some specific embodiments of the present disclosure, the step of mixing the hyaluronic acid solution with the liposome solution to form the hyaluronic acid liposome assembly comprises: adding the hyaluronic acid solution to the liposome solution at 20-40 °C, uniformly mixing a resulting solution to form a stable solution, then removing the solvent used for preparing the liposome solution, adding a buffer solution, and uniformly mixing the solution to obtain the hyaluronic acid liposome assembly.

According to some specific embodiments of the present disclosure, the step of mixing the hyaluronic acid solution with the liposome solution to form the hyaluronic acid liposome assembly comprises adding the hyaluronic acid solution to the liposome solution at 25-35 °C.

According to some specific embodiments of the present disclosure, the step of mixing the liposome solution with the hyaluronic acid solution to form the hyaluronic acid liposome assembly comprises adding the hyaluronic acid solution to the liposome solution at 25-30 °C.

According to some specific embodiments of the present disclosure, the step of adding the hyaluronic acid solution to the liposome solution under stirring condition to form the hyaluronic acid liposome assembly comprises: adding the hyaluronic acid solution to the liposome solution at a rate of 1-25 mL/min, uniformly mixing a resulting solution to form a stable solution, then removing the solvent used for preparing the liposome solution, adding a buffer solution or water, and uniformly mixing the solution to obtain the hyaluronic acid liposome assembly.

According to some specific embodiments of the present disclosure, the step of adding the hyaluronic acid solution to the liposome solution under stirring condition to form the hyaluronic acid liposome assembly comprises adding the hyaluronic acid solution to the liposome solution at a rate of 5-25 mL/min.

For the step of "adding a buffer solution or water, and uniformly mixing the solution", stirring, shaking, or ultrasonication can be used for uniform mixing.

According to some specific embodiments of the present disclosure, in the step of forming the hyaluronic acid liposome assembly, the stirring condition for adding the hyaluronic acid solution to the liposome solution under stirring condition includes a stirring time of 5-30 min.

According to some specific embodiments of the present disclosure, the stirring condition includes a stirring time of 5-20 min.

It can be understood that the stirring time described here starts from the beginning of adding the hyaluronic acid solution to the end of stirring for uniform mixing to form a stable solution.

According to some specific embodiments of the present disclosure, in the step of forming the hyaluronic acid liposome assembly, after the hyaluronic acid solution is added to the liposome solution, stirring is continued at an original rotation speed to form a stable solution.

According to some specific embodiments of the present disclosure, in the step of forming the hyaluronic acid liposome assembly, after the hyaluronic acid solution is added to the liposome solution, stirring is carried out at 30-50 °C to form a stable solution.

According to some specific embodiments of the present disclosure, in the step of forming the hyaluronic acid liposome assembly, after the hyaluronic acid solution is added to the liposome solution, stirring is carried out at 35-45 °C to form a stable solution.

According to some specific embodiments of the present disclosure, in the step of forming the hyaluronic acid liposome assembly, after the hyaluronic acid solution is added to the liposome solution, a resulting solution is uniformly mixed by a high-shear dispersion emulsifying machine to form a stable solution.

According to some specific embodiments of the present disclosure, in the step of removing the solvent used for preparing the liposome solution, the solvent used for preparing the liposome solution is removed by rotary evaporation.

According to some specific embodiments of the present disclosure, an evaporation bottle of the rotary evaporator used for the rotary evaporation has a rotation speed of 80-120 rpm, and a temperature of 40-70 °C.

According to some specific embodiments of the present disclosure, an evaporation bottle of the rotary evaporator used for the rotary evaporation has a rotation speed of 80-100 rpm, and a temperature of 45-60 °C.

According to some specific embodiments of the present disclosure, an evaporation bottle of the rotary evaporator r used for the rotary evaporation has a degree of vacuum of 70-100 kPa.

According to some specific embodiments of the present disclosure, an evaporation bottle of the rotary evaporator used for the rotary evaporation has a degree of vacuum of 80-95 kPa.

The temperature described above may be either the temperature of the stable solution or the temperature of the water bath for rotary evaporation (after rotary evaporation for a certain time, the temperature of the stable solution in a container should be approximately the same as the temperature of the water bath).

According to some specific embodiments of the present disclosure, the step of forming the hyaluronic acid liposome assembly further comprises: after the solvent used for preparing the liposome solution is removed, adding a buffer solution or water, uniformly mixing a resulting solution, and then passing the uniform mixed solution through an 800 nm filter membrane to form a primary hyaluronic acid liposome assembly; then adding a buffer solution or water to achieve a stable state and then passing the solution through a 450 nm filter membrane; and repeating the above operation starting from passing the uniform mixed solution through an 800 nm filter membrane for 0-2 times to obtain the hyaluronic acid liposome assembly.

According to some specific embodiments of the present disclosure, the filter membrane is a polycarbonate filter membrane, a polytetrafluoroethylene filter membrane, a polypropylene filter membrane, or a polyvinylidene fluoride filter membrane.

It can be understood that the phrase of "the above operation starting from passing the uniform mixed solution through an 800 nm filter membrane for 0-2 times" as described above means repeating the process of "passing the uniform mixed solution through an 800 nm filter membrane to form a primary hyaluronic acid liposome assembly; then adding a buffer solution or water to achieve a stable state and then passing the solution through a 450 nm filter membrane", and preferably, the process is repeated once or twice.

It can also be understood that the product obtained before the last passage through the 450 nm filter membrane is referred to as the primary hyaluronic acid liposome assembly, and the product obtained after the last passage through the 450 nm filter membrane is the hyaluronic acid liposome assembly.

According to some specific embodiments of the present disclosure, in the step of forming the hyaluronic acid liposome assembly, on the basis of an amount of the phospholipid, an addition amount of the buffer solution or water each time is separately and independently 8-12 mL of the buffer solution or water for every 0.1 g of the phospholipid.

According to some specific embodiments of the present disclosure, in the step of forming the hyaluronic acid liposome assembly, on the basis of an amount of the phospholipid, an addition amount of the buffer solution or water each time is separately and independently 10 mL of the buffer solution or water for every 0.1 g of the phospholipid.

According to some specific embodiments of the present disclosure, in the step of forming the hyaluronic acid liposome assembly, a mass percentage content of a primary hyaluronic acid liposome in a solution obtained before the last passage through the 450 nm filter membrane is 45%-65%, and a mass percentage content of a hyaluronic acid liposome assembly in a solution obtained after the passage through the 450 nm filter membrane is 25%-35%.

According to some specific embodiments of the present disclosure, in the step of forming the hyaluronic acid liposome assembly which comprises passing the uniform mixed solution through an 800 nm polycarbonate membrane to form a primary hyaluronic acid liposome assembly, and then adding a buffer solution or water to achieve a stable state, the stable state is achieved by stirring at a rotation speed of 10,000-20,000 for 5-30 min.

According to some specific embodiments of the present disclosure, in the step of forming the hyaluronic acid liposome assembly, the stable state is achieved by stirring at a rotation speed of 10,000-15,000 for 10-25 min.

According to some specific embodiments of the present disclosure, in the step of forming the hyaluronic acid liposome assembly, the stable state is achieved by uniform mixing by a high-shear dispersion emulsifying machine.

According to some specific embodiments of the present disclosure, the buffer solution is separately and independently a phosphate buffer solution with a concentration of 1-10 mg/mL.

In a further aspect, the present disclosure further provides a hyaluronic acid liposome assembly prepared by the preparation method described in the present disclosure.

In yet another aspect, the present disclosure further provides use of the hyaluronic acid liposome assembly as described in any aspect of the present disclosure in the preparation of a transdermal absorption product.

According to some specific embodiments of the present disclosure, the transdermal absorption product is a transdermal absorption drug or a transdermal absorption skin care product.

In summary, the present disclosure provides a hyaluronic acid liposome assembly, a preparation method, and use thereof. The hyaluronic acid liposome assembly of the present disclosure has the following advantages.
(1) Compared with traditional liposomes, hyaluronic acids with different molecular weights are used in the present disclosure to construct liposomes, thus forming stable hyaluronic acid liposome assemblies (HA-Liposome assemblies). Under the skin-moisturizing and permeation-enhancing dual effects of the hyaluronic acid, the hyaluronic acid further synergistically work with the liposome to achieve an effect of efficient permeation of the hyaluronic acid liposome assembly into the skin.
(2) Hyaluronic acid (HA), as a scaffold molecule, participates in a formation of the liposome assembly structure, instead of being wrapped by the liposome. This structure is beneficial to the adaptive deformation of hyaluronic acid with the liposome, which is beneficial to transdermal delivery.
(3) The hyaluronic acid and the liposome can both directly enter the skin directly through cell pathways or gaps. Furthermore, the intercellular gaps in the stratum corneum of the skin can become swollen and fluffy, thereby achieving the effect of enhancing the absorption of small molecular drugs. Therefore, the combined use of the hyaluronic acid and the liposome is expected to achieve a synergistic purpose of transdermal delivery.
(4) The existing market perception has been challenged because in the hyaluronic acid liposome assemblies constructed in the present disclosure, which have hyaluronic acids with four different molecular weights, the hyaluronic acids with high molecular weights can efficiently permeate, and the hyaluronic acids with high molecular weights provide the best moisturizing and repairing effects on the skin and have a relatively long metabolic degradation time.
(5) In the present disclosure, the hyaluronic acids with different molecular weights are used to participate in the construction of liposome, and a series of hyaluronic acid liposome assemblies having an effect of efficient skin permeation are prepared. Such assemblies can be used as transdermal drug delivery carriers and can achieve the purpose of effective loading of various types of drugs and efficient skin permeation.
(6) The selected components are all materials approved by the China Food and Drug Administration for use as pharmaceutically acceptable excipients and have good biological safety.
(7) The hyaluronic acid liposome assembly can not only realize efficient transdermal delivery of the hyaluronic acid itself, but can also load other active small molecules, so as to realize the efficient transdermal delivery of the loaded active ingredient.

### BRIEF DESCRIPTION OF DRAWINGS

FIG. 1 is a schematic structural diagram of an existing hyaluronic acid liposome assembly and a hyaluronic acid liposome assembly of the present disclosure;
FIGs. 2A and 2B show the particle size distributions of the hyaluronic acid liposome assemblies with hyaluronic acids having different molecular weights descried in Example 1;
FIGs. 3A and 3B are respectively the fluorescence morphologies of hyaluronic acid liposome assemblies with hyaluronic acids having different molecular weights descried in Examples 3 and 4;
FIGs. 4A and 4B are respectively the skin permeation effects of the hyaluronic acid liposome assemblies with hyaluronic acids having different molecular weights descried in Examples 3 and 4 on mice;
FIG. 5 is fluorescence skin sections after an in vitro transdermal experiment of the hyaluronic acid liposome assemblies with hyaluronic acids having different molecular weights; and
FIG. 6 shows cytotoxicities of the hyaluronic acid liposome assemblies with hyaluronic acids having different molecular weights.

### DETAILED DESCRIPTION

Specific examples are used below to explain in detail the implementation process and beneficial effects of the present disclosure, aiming to help readers better understand the essence and features of the present disclosure, and are not limitations on the scope of implementation of the present disclosure.

### Example 1

a. At 25 °C, soybean lecithin and cholesterol were dissolved in a certain amount of ethanol at a certain proportion, stirred and dissolved by a magnetic heating stirrer (or dissolved by a low-speed homogenizer appropriately), and after being fully dissolved, the solution was stabilized for 2 h to obtain an alcohol liposome suspension; wherein a mass ratio of cholesterol to soybean lecithin was 1:10; an amount of ethanol was based on every 10 mL ethanol corresponding to 0.1 g of the soybean lecithin; a purity of the soybean lecithin was ≥ 90%; a purity of the cholesterol was 95%; the ethanol was industrial grade, with a purity of 99%; and a temperature of the magnetic heating stirrer was 35 °C, a rotation speed was 1500 rpm, and a stirring time was 20 min.
b. At 25 °C, a series of hyaluronic acids with different molecular weights (with an amount of 25 mg each) were respectively dissolved in a certain amount of a phosphate buffer solution, fully dissolved by a vortex shaker (or dissolved by a low-speed homogenizer appropriately), and then stabilized for 3 h; wherein the molecular weight distributions of the series of hyaluronic acids with different molecular weights were 3000 (model number J44455), 150,000-250,000 (model number J44456), 400,000-1,000,000 (model number J36310), and 1,000,000-1,500,000 (model number J31234), respectively; an amount of the phosphate buffer solution was based on every 10 mL phosphate buffer solution corresponding to 15 mg of the hyaluronic acid with different molecular weights; a temperature of the vortex shaker was 25 °C, and a rotation speed was 1,000 rpm; and a pH value of the phosphate buffer solution was 7.4.
c. At 25 °C, the alcohol liposome suspension obtained in step a was stirred with a magnetic heating stirrer, and each of the hyaluronic acid buffer solution obtained in step b was slowly added to the alcohol liposome suspension obtained in step a at a rate of 5 mL/min and stirred and dissolved by a high-shear dispersion emulsifying machine until a stable emulsion was formed; ethanol was removed from the emulsion at a certain temperature and pressure by a rotary evaporator, the temperature was lowered to room temperature, and a certain amount of the phosphate buffer solution was added and homogeneously mixed until uniform; a resulting hyaluronic acid liposome assembly was passed through a 800 nm polycarbonate membrane to form a primary hyaluronic acid liposome assembly, the primary hyaluronic acid liposome assembly was slowly added to a certain amount of the phosphate buffer solution and stirred to a stable state by a high-shear dispersion emulsifying machine, then passed through a 450 nm polycarbonate membrane, and this operation was repeated twice to finally obtain a stable final hyaluronic acid liposome assembly. A mass ratio of the hyaluronic acids with different molecular weights in step b to the soybean lecithin in step a was 1:40; a temperature of the magnetic heating stirrer was 45 °C, a rotation speed was 1,000 rpm, and a stirring time was 20 min; the phosphate buffer solution had a pH of 7.4 and could be replaced with pure water; a volume ratio of the phosphate buffer solution to the phosphate buffer solution in step b was 1:1.5; a power of the high-shear dispersion emulsifying machine was 300 W, a rotation speed was 10,000 rpm, and the high-shear dispersion emulsifying machine was operated in each instance for 25 min; a rotation speed of an evaporation bottle of the rotary evaporator was 100 rpm, ta degree of vacuum was 80 kPa, and a temperature of a thermostat water bath was 60 °C; a mass percentage of the hyaluronic acid liposome assembly in the primary hyaluronic acid liposome assembly was 65%; and a mass percentage of the hyaluronic acid liposome assembly in the final hyaluronic acid liposome assembly was 45%.

### Example 2

a. At 30 °C, soybean lecithin and cholesterol were dissolved in a certain amount of ethanol at a certain proportion, stirred and dissolved by a magnetic heating stirrer (or dissolved by a low-speed homogenizer appropriately), and after being fully dissolved, the solution was stabilized for 6 h to obtain an alcohol liposome suspension; wherein a mass ratio of cholesterol to soybean lecithin was 1:25; an amount of ethanol was based on every 10 mL ethanol corresponding to 0.1 g of the soybean lecithin; a purity of the soybean lecithin was ≥ 90%; a purity of the cholesterol was 95%; the ethanol was industrial grade, with a purity of 99%; and a temperature of the magnetic heating stirrer was 45 °C, a rotation speed was 1,000 rpm, and a stirring time was 10 min.
b. At 30 °C, a series of hyaluronic acids with different molecular weights (with an amount of 25 mg each) were respectively dissolved in a certain amount of a phosphate buffer solution, fully dissolved by a vortex shaker (or dissolved by a low-speed homogenizer appropriately), and then stabilized for 3 h; wherein the molecular weight distributions of the series of hyaluronic acids with different molecular weights were 3000 (model number J44455), 150,000-250,000 (model number J44456), 400,000-1,000,000 (model number J36310), and 1,000,000-1,500,000 (model number J31234), respectively; an amount of the phosphate buffer solution was based on every 10 mL phosphate buffer solution corresponding to 50 mg of the hyaluronic acid with different molecular weights; a temperature of the vortex shaker was 35 °C, and a shaking time in each instance was 5 min, and a rotation speed was 1,000 rpm; and a pH value of the phosphate buffer solution was 7.4.
c. At 30 °C, the alcohol liposome suspension obtained in step a was stirred with a magnetic heating stirrer, and each of the hyaluronic acid buffer solution obtained in step b was slowly added to the alcohol liposome suspension obtained in step a at a rate of 25 mL/min and stirred and dissolved by a high-shear dispersion emulsifying machine until a stable emulsion was formed; ethanol was removed from the emulsion by a rotary evaporator, the temperature was lowered to room temperature, and a certain amount of the phosphate buffer solution was added and homogeneously mixed until uniform; a resulting hyaluronic acid liposome assembly was passed through a 800 nm polycarbonate membrane to form a primary hyaluronic acid liposome assembly, the primary hyaluronic acid liposome assembly was slowly added to a certain amount of the phosphate buffer solution and stirred to a stable state by a high-shear dispersion emulsifying machine, then passed through a 450 nm polycarbonate membrane, and this operation was repeated three times finally obtain a stable final hyaluronic acid liposome assembly. A mass ratio of the hyaluronic acids with different molecular weights in step b to the soybean lecithin in step a was 1:100; a temperature of the magnetic heating stirrer was 35 °C, a rotation speed was 2000 rpm, and a stirring time was 5 min; the phosphate buffer solution had a pH of 7.4 and could be replaced with pure water; a volume ratio of the phosphate buffer solution to the phosphate buffer solution in step b was 1:4; a power of the high-shear dispersion emulsifying machine was 500 W, a rotation speed was 15000 rpm, and the high-shear dispersion emulsifying machine was operated in each instance for 10min; a rotation speed of an evaporation bottle of the rotary evaporator was 80 rpm, a degree of vacuum was 95 kPa, and a temperature of a thermostat water bath was 45 °C; a mass percentage of the hyaluronic acid liposome assembly in the primary hyaluronic acid liposome assembly was 65%; and a mass percentage of the hyaluronic acid liposome assembly in the final hyaluronic acid liposome assembly was 45%.

### Example 3

In order to intuitively observe and accurately determine the assembly process of the hyaluronic acid liposomes obtained in Examples 1 and 2 and the content of the hyaluronic acid, we used 5-aminofluorescein to perform fluorescence labeling at the maximum saturation level for the hyaluronic acids with different molecular weights of Examples 1 and 2. The specific experimental steps were as shown in Examples 3 and 4:
a. At 25 °C, soybean lecithin and cholesterol were dissolved in a certain amount of ethanol at a certain proportion, stirred and dissolved by a magnetic heating stirrer (or dissolved by a low-speed homogenizer appropriately), and after being fully dissolved, the solution was stabilized for 2 h to obtain an alcohol liposome suspension, wherein a mass ratio of cholesterol to soybean lecithin was 1:10; an amount of ethanol was based on every 10 mL ethanol corresponding to 0.1 g of the soybean lecithin; a purity of the soybean lecithin was ≥ 90%; a purity of the cholesterol was 95%; the ethanol was industrial grade, with a purity of 99%; and a temperature of the magnetic heating stirrer was 35 °C, a rotation speed was 1500 rpm, and a stirring time was 20 min.
b. At 25 °C, 1-ethyl-(3-dimethylaminopropyl)carbodiimide hydrochloride (EDC-HCl) and N-hydroxysuccinimide (NHS) were added to each of the aqueous solution of the hyaluronic acids with different molecular weights, respectively, the reaction was stirred for 4 h in the dark by a magnetic heating stirrer, 5-aminofluorescein was then added, the reaction was continued for 24 h, and then, after dialysis with a 1,000 Da dialysis bag until no fluorescein was present, freeze-drying was carried out. A molar ratio of hyaluronic acid to EDC-HCl to NHS to 5-aminofluorescein was 1:1:1.2:1.5; an amount of pure water was based on every 10 mL pure water corresponding to 10 mg of the hyaluronic acids with different molecular weights; a temperature of the magnetic heating stirrer was 25 °C, and a rotation speed was 1,000 rpm; and no fluorescein being present during dialysis by the dialysis bag was determined by the criterion that an absorbance of the dialysate was less than 0.06 at 486 nm by an ultraviolet-visible spectrophotometer, by which no fluorescein precipitation in the dialysate could be determined.
c. At 25 °C, the 5-aminofluorescein-labeled hyaluronic acids with different molecular weights as obtained above in step b (with an amount of 25 g each) were respectively dissolved in a certain amount of a phosphate buffer solution by a magnetic heating stirrer, fully dissolved, and stabilized for 3 h; wherein the molecular weights of the series of hyaluronic acids with different molecular weights were 3000 (model number J44455), 150,000-250,000 (model number J44456), 400,000-1,000,000 (model number J36310), and 1,000,000-1,500,000 (model number J31234), respectively; an amount of the phosphate buffer solution was based on every 10 mL phosphate buffer solution corresponding to 15 mg of the 5-aminofluorescein-labeled hyaluronic acids with different molecular weights; a temperature of the magnetic heating stirrer was 25 °C, and a rotation speed was 1,000 rpm; and a pH value of the phosphate buffer solution was 7.4.
d. At 25 °C, the alcohol liposome suspension obtained in step a was stirred with a magnetic heating stirrer, and each of the hyaluronic acid buffer solution obtained in step c was slowly added to the alcohol liposome suspension obtained in step a at a rate of 5 mL/min and stirred and dissolved by a high-shear dispersion emulsifying machine until a stable emulsion was formed; ethanol was removed from the emulsion by a rotary evaporator, the temperature was lowered to room temperature, and a certain amount of the phosphate buffer solution was added and homogeneously mixed until uniform; a resulting hyaluronic acid liposome assembly was passed through a 800 nm polycarbonate membrane to form a primary hyaluronic acid liposome assembly, the primary hyaluronic acid liposome assembly was slowly added to a certain amount of the phosphate buffer solution and stirred to a stable state by a high-shear dispersion emulsifying machine, then passed through a 450 nm polycarbonate membrane, and this operation was repeated twice to finally obtain a stable final hyaluronic acid liposome assembly A mass ratio of the 5-aminofluorescein-labeled hyaluronic acids with different molecular weights obtained in step b to the soybean lecithin in step a was 1:40; a temperature of the magnetic heating stirrer was 45 °C, a rotation speed was 1,000 rpm, and a stirring time was 20 min; the phosphate buffer solution could be replaced with pure water; a power of the high-shear dispersion emulsifying machine was 300 W, a rotation speed was 10,000 rpm, and the high-shear dispersion emulsifying machine was operated in each instance for 25 min; a rotation speed of an evaporation bottle of the rotary evaporator was 100 rpm, a degree of vacuum was 80 kPa, and a temperature of a thermostat water bath was 60 °C; a mass percentage of the hyaluronic acid liposome assembly in the primary hyaluronic acid liposome assembly was 65%; and a mass percentage of the hyaluronic acid liposome assembly in the final hyaluronic acid liposome assembly was 45%.

### Example 4

a. At 30 °C, soybean lecithin and cholesterol were dissolved in a certain amount of ethanol at a certain proportion, stirred and dissolved by a magnetic heating stirrer (or a low-speed homogenizer), and after being fully dissolved, the solution was stabilized for 6 h to obtain an alcohol liposome suspension; wherein a mass ratio of cholesterol to soybean lecithin was 1:25; an amount of ethanol was based on every 10 mL ethanol corresponding to 0.1 g of the soybean lecithin; a purity of the soybean lecithin was ≥ 90%; a purity of the cholesterol was 95%; the ethanol was industrial grade, with a purity of 99%; and a temperature of the magnetic heating stirrer was 45 °C, a rotation speed was 1,000 rpm, and a stirring time was 10 min.
b. At 30 °C, 1-ethyl-(3-dimethylaminopropyl)carbodiimide hydrochloride (EDC-HCl) and N-hydroxysuccinimide (NHS) were added to each of the aqueous solution of the hyaluronic acids with different molecular weights, respectively, the reaction was stirred for 8 h in the dark by a magnetic heating stirrer, 5-aminofluorescein was then added, the reaction was continued for 48 h, and then, after dialysis with a 1,000 Da dialysis bag until no fluorescein was present, freeze-drying was carried out. A molar ratio of hyaluronic acid to EDC-HCl to NHS to 5-aminofluorescein was 1:1.5:2:2.5; an amount of pure water was based on every 10 mL pure water corresponding to 5 mg of the hyaluronic acids with different molecular weights; a temperature of the magnetic heating stirrer was 35 °C, and a rotation speed was 1,000 rpm; and no fluorescein being present during dialysis by the dialysis bag was determined by the criterion that an absorbance of the dialysate was less than 0.06 at 486 nm by an ultraviolet-visible spectrophotometer, by which no fluorescein precipitation in the dialysate could be determined.
c. At 30 °C, the 5-aminofluorescein-labeled hyaluronic acids with different molecular weights obtained above in step b (with an amount of 25 mg each) were respectively dissolved in a certain amount of a phosphate buffered solution, fully dissolved by a vortex shaker (or a homogenizer), and then stabilized for 3 h; wherein the molecular weight distributions of the 5-aminofluorescein-labeled hyaluronic acids with different molecular weights were 3000 (model number J44455), 150,000-250,000 (model number J44456), 400,000-1,000,000 (model number J36310), and 1,000,000-1,500,000 (model number J31234); an amount of the phosphate buffer solution was based on every 10 mL phosphate buffer solution corresponding to 50 mg of the 5-aminofluorescein-labeled hyaluronic acids with different molecular weights; a temperature of the vortex shaker was 35 °C, and a time for each instance of shaking was 5 min, and a rotation speed was 1,000 rpm; and the phosphate buffered solution had a pH of 7.4.
d. At 30 °C, the alcohol liposome suspension obtained in step a was stirred with a magnetic heating stirrer, and each of the buffer solution of the 5-aminofluorescein-labeled hyaluronic acids with different molecular weights obtained in step c was slowly added to the alcohol liposome suspension obtained in step a at a rate of 25 mL/min and stirred and dissolved by a high-shear dispersion emulsifying machine until a stable emulsion was formed; ethanol was removed from the emulsion by a rotary evaporator, the temperature was lowered to room temperature, and a certain amount of the phosphate buffer solution was added and homogeneously mixed until uniform; a resulting hyaluronic acid liposome assembly was passed through a 800 nm polycarbonate membrane to form a primary hyaluronic acid liposome assembly, and the primary hyaluronic acid liposome assembly was slowly added to a certain amount of the phosphate buffer solution and stirred to a stable state by a high-shear dispersion emulsifying machine, then passed through a 450 nm polycarbonate membrane, and this operation was repeated three times ally obtain a stable final hyaluronic acid liposome assembly. A mass ratio of the 5-aminofluorescein-labeled hyaluronic acids with different molecular weights obtained in step b to the soybean lecithin in step a was 1:100;a temperature of the magnetic heating stirrer was 35 °C, a rotation speed was 2000 rpm, and a stirring time was 5 min; the phosphate buffer solution had a pH of 7.4 and could be replaced with pure water; a volume ratio of the phosphate buffer solution to the phosphate buffer solution in step b was 1:4; a power of the high-shear dispersion emulsifying machine was 500 W, a rotation speed was 15000 rpm, and the high-shear dispersion emulsifying machine was operated in each instance for 10min; a rotation speed of an evaporation bottle of the rotary evaporator was 80 rpm, a degree of vacuum was 95 kPa, and a temperature of a thermostat water bath was 45 °C; a mass percentage of the hyaluronic acid liposome assembly in the primary hyaluronic acid liposome assembly was 65%; and a mass percentage of the hyaluronic acid liposome assembly in the final hyaluronic acid liposome assembly was 45%.

### Example 5

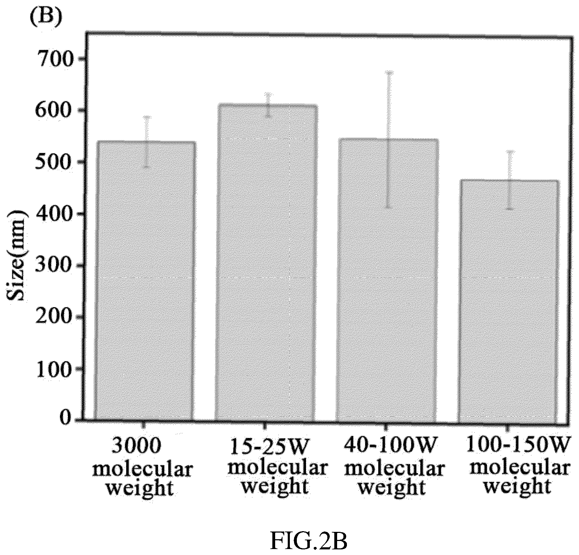

After the series of hyaluronic acid liposome assemblies with hyaluronic acids having different molecular weights as obtained in Example 1 were diluted into the same concentration, the morphologies thereof were observed by a microscope, and the particle sizes thereof were further determined by Malvern particle size analyzer. The experimental results were as shown in FIGs. 2A and 2B. It can be seen intuitively by the microscope that the hyaluronic acid liposome assemblies with hyaluronic acids having different molecular weights had uniform particle sizes and good dispersibility, and the particle size distributions were about 500 nm as determined by the particle size analyzer.

### Example 6

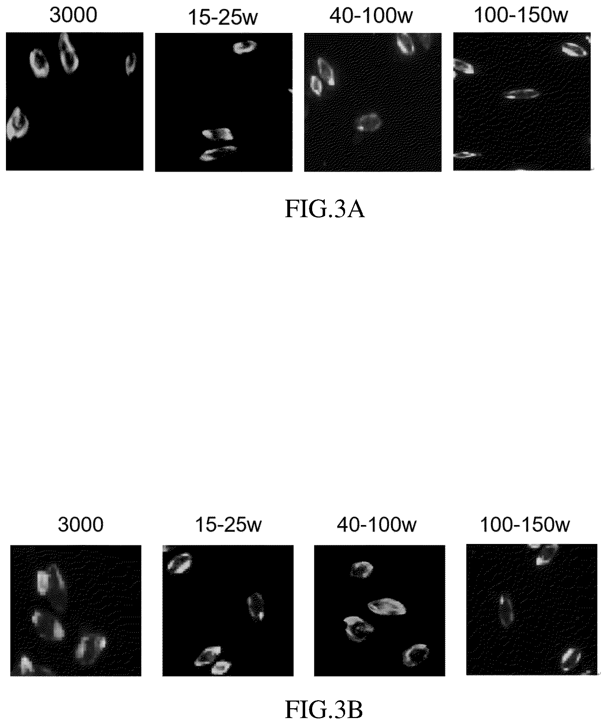

The 5-aminofluorescein-labeled hyaluronic acid liposome assemblies with hyaluronic acids having different molecular weights as obtained in Examples 3 and 4 were observed by a fluorescence microscope. The experimental results were as shown in FIGs. 3A and 3B. With the increase of the molecular weight of the hyaluronic acid, we found that the fluorescently labeled hyaluronic acids were uniformly filled in the liposome and formed a stable ring structure with the liposome. Especially for the hyaluronic acid with a high molecular weight of 1,000,000-1,500,000, the ring structure of the hyaluronic acid liposome assembly was more obvious. Therefore, we believe that the hyaluronic acid can effectively participate in the construction of liposome and can be assembled with the liposome to form a stable cavity ring structure. FIGs. 3A and 3B demonstrated that the hyaluronic acid was involved inside the scaffold of the liposome, instead of traditionally being wrapped by liposome. If the hyaluronic acid is wrapped by the liposome, the hyaluronic acid should be a green (while light gray was shown in FIGs. 3A and 3B) spherical structure or the center of the ring shows obvious green fluorescence (while light gray or white color was shown in FIGs. 3A and 3B).

### Example 7

The 5-aminofluorescein-labeled hyaluronic acid liposome assemblies with hyaluronic acids having different molecular weights as obtained in Examples 3 and 4 were subjected to a skin permeability test in vitro by Franz transdermal diffusion cells. The specific operation was as follows:
Mice (purchased from Beijing HFK Bioscience Co., Ltd., China) were anesthetized by intraperitoneal injection of pentobarbital sodium (40 mg/kg) and then sacrificed by cervical dislocation, the hair on the back of the mice was removed by an electric razor, and the intact skin from which the hair had been removed was then cut off by scissors. The dermal layer of the skin was wiped with a cotton ball dipped with normal saline, the adhered subcutaneous tissue was removed, the skin was then washed with normal saline, wiped dry, wrapped with a tin foil, and stored in a refrigerator at -20 °C for later use. The prepared skin was fixed between a supply chamber and a receiving chamber of the Franz diffusion cell, wherein the surface layer of the skin faced the supply chamber, and an effective permeation area of the diffusion cell was 1.767 cm². 10 mL of a phosphate buffer solution (PBS, pH = 7.4) was added to the receiving chamber, such that the liquid surface could be in close contact with the skin. During the experiment, a circulating water bath was used for heating such that the temperature was maintained at 37 °C, and stirring was carried out at a speed of 150 r/min.

2 mL of the 5-aminoluorescein-labeled hyaluronic acid liposome assemblies with hyaluronic acids having different molecular weights as obtained in Example 3 with the same concentration (the initial hyaluronic acid concentration was recorded) were respectively taken by a pipette and added to supply chambers of diffusion cells. Then, equal volumes of lower sample solutions were drawn from a sampling port within the same time, and at the same time equal volumes of PBS were added to replenish the receiving chambers. The samples were quantitatively analyzed by a fluorescence spectrophotometer, and the cumulative amount of the hyaluronic acid liposome permeating the skin over time (the sum of the hyaluronic acid inside the skin and that permeating the skin) was derived by calculation. After the experiment was completed, the skin was embedded in paraffin and made into sections, and the liposome distribution inside the skin was observed.

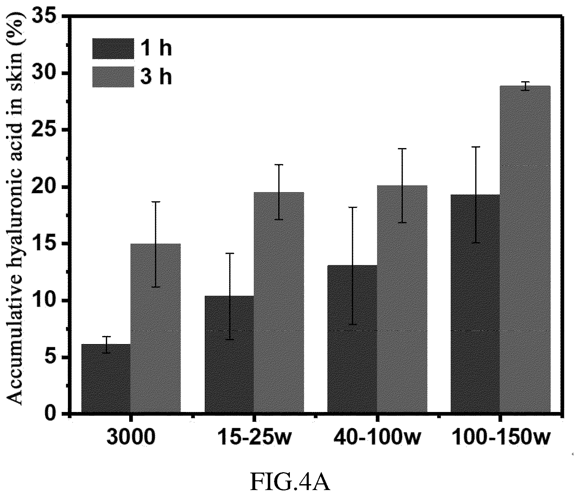

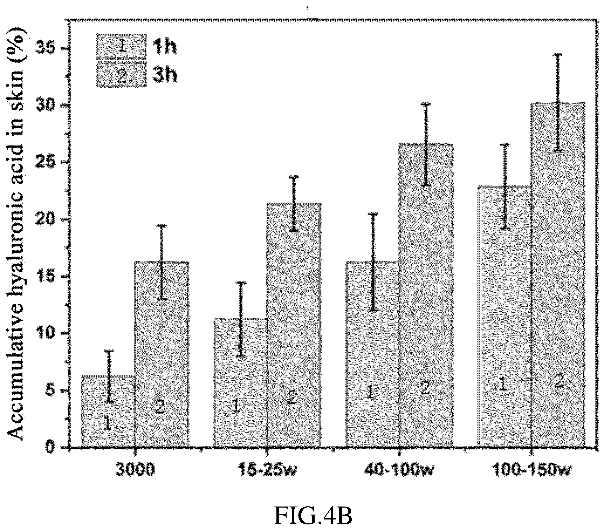

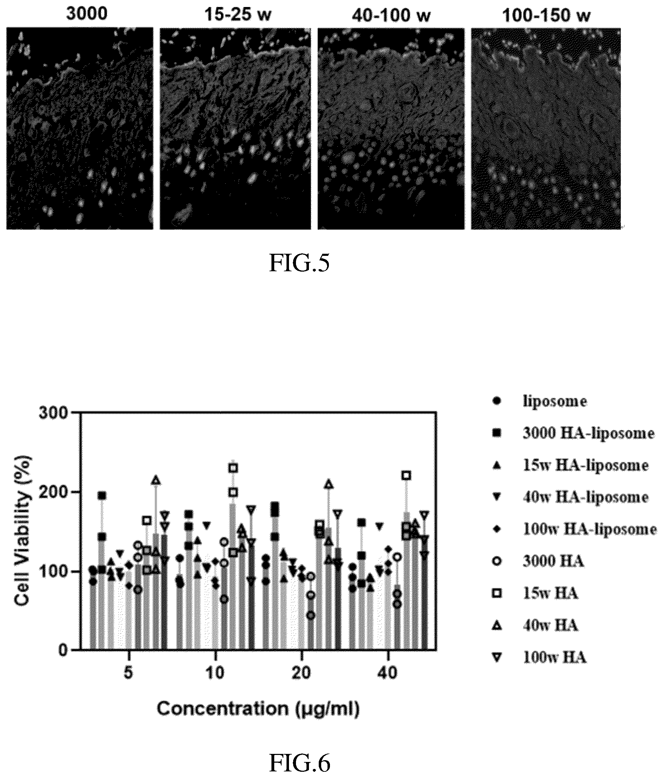

The results were as shown in FIGs. 4A and 4B, as could be seen, after 1 to 3 h, the hyaluronic acid liposome assemblies with hyaluronic acids having different molecular weights all had certain transdermal effects, and with the increase of the molecular weight of the hyaluronic acid, the transdermal effect of the liposome also significantly increased, and the accumulative content of the hyaluronic acid in the skin also increased. After calculation, the hyaluronic acid liposome assemblies with hyaluronic acid having molecular weight of 1,000,000-1,500,000 obtained in Examples 3 and 4 had the best skin permeation effect, and the accumulative content of the hyaluronic acid in the skin could reach about 20% and could reach about 30% at 3 h. Therefore, we found that the hyaluronic acid liposome assemblies could effectively achieve efficient permeation of the hyaluronic acids with high molecular weights into the skin. In addition, we observed the distribution of the hyaluronic acid liposomes inside the skin by a fluorescence microscope. The experimental results were as shown in FIG. 5. We could intuitively find the presence of the fluorescently labeled hyaluronic acid liposomes in different structural parts of the skin, such as the surface and inside of the skin and below the dermal layer, and for the hyaluronic acid liposome assembly with hyaluronic acid having molecular weight of 1,000,000-1,500,000, it can be clearly found that a large number of fluorescently labeled liposomes had completely permeated the dermal layer of the skin. Therefore, the above results have fully explained that the hyaluronic acid liposome assembly could achieve effective permeation of the hyaluronic acids with different molecular weights into the skin, and the effect was the best when the molecular weight was 1,000,000-1,500,000.

### Example 8

The series of hyaluronic acid liposome assemblies with hyaluronic acids having different molecular weights as prepared in Example 1 were filtered, sterilized, and then prepared into solutions in a certain concentration gradient (5, 10, 20, and 40 g/mL) with a medium, which were added to rat fibroblasts (purchased from Guangzhou Diyi Junqu Yiyuan, China) with a confluence of 70% for incubation. After 24 h, the cytotoxicity of the material was determined by a CCK-8 method. The results were as shown in FIG. 6, as could be seen, when the sample concentration reached 40 g/mL, the cell survival rate remained 95% or more, and a certain phenomenon of enhancing the cell growth was found. Compared with the cell survival rate in groups of pure hyaluronic acid and pure liposome, the cell survival rate in the hyaluronic acid liposome assembly was close, and there was no significant difference. The results of FIG. 6 showed that the hyaluronic acid liposome assemblies with hyaluronic acids having different molecular weights did not change the good biocompatibility of the hyaluronic acids.

## Claims

1. A hyaluronic acid liposome assembly comprising hyaluronic acid and liposome at a mass ratio of 1:20-150, (preferably hyaluronic acid and liposome at a mass ratio of 1:40-100); wherein the hyaluronic acid is located in a bimolecular layer of the liposome and constitutes the bimolecular layer of the liposome with a hyaluronic acid scaffold structure; and a mass of the liposome is based on a mass of phospholipid in the liposome.

2. The hyaluronic acid liposome assembly according to claim 1, wherein the hyaluronic acid has a molecular weight of 3000-1,500,000 (preferably, the hyaluronic acid is at least one of or a combination of some of hyaluronic acids having the following molecular weight ranges: a hyaluronic acid with a molecular weight of 3000, a hyaluronic acid with a molecular weight of 150,000-250,000, a hyaluronic acid with a molecular weight of 400,000-1,000,000, and a hyaluronic acid with a molecular weight of 1,000,000-1,500,000).

3. The hyaluronic acid liposome assembly according to claim 1 or 2, wherein the liposome is composed of cholesterol and phospholipid (preferably, the phospholipid is one or more of soybean lecithin, dioleoyl lecithin, and egg yolk lecithin) at a mass ratio of 1:5-30 (preferably, the liposome is composed of cholesterol and phospholipid at a mass ratio of 1:10-25).

4. A method for preparing the hyaluronic acid liposome assembly according to any one of claims 1 to 3, comprising the following steps:
preparing a liposome solution;
preparing a hyaluronic acid solution; and
adding the hyaluronic acid solution to the liposome solution under stirring condition (preferably a stirring speed is 1000-5000 rpm, more preferably 1000-2000 rpm) to form the hyaluronic acid liposome assembly.

5. The method according to claim 4, wherein the step of preparing the liposome solution comprises dissolving liposome in a solvent (preferably, a solvent used for dissolving the liposome is ethanol, diethyl ether, or chloroform) to obtain the liposome solution.

6. The method according to claim 5, wherein on the basis of the phospholipid in the liposome, an amount of the solvent used for dissolving the liposome is that every 10 mL of the solvent dissolves 0.1-0.8 g (preferably 0.1-0.5 g) of the phospholipid.

7. The method according to claim 5 or 6, wherein the liposome solution is obtained by stabilization for 1.5-10 h (preferably 2-6 h) after the liposome is dissolved in the solvent.

8. The method according to any one of claims 4 to7, wherein the step of preparing the hyaluronic acid solution comprises dissolving hyaluronic acid in a buffer solution or water until uniformly dissolved to obtain the hyaluronic acid solution.

9. The method according to claim 8, wherein on the basis of the hyaluronic acid, an amount of the buffer solution is that every 10 mL of the buffer solution dissolves 10-100 mg of the hyaluronic acid (preferably 13-60 mg of the hyaluronic acid).

10. The method according to claim 8 or 9, wherein the hyaluronic acid is dissolved in the buffer solution until uniformly dissolved and then stabilized for 2-10 h (preferably 3-7 h) to obtain the hyaluronic acid solution.

11. The method according to any one of claims 4 to 10, wherein the step of adding the hyaluronic acid solution to the liposome solution under stirring condition to form the hyaluronic acid liposome assembly comprises: adding the hyaluronic acid solution to the liposome solution at a rate of 1-25 mL/min under stirring condition, uniformly mixing a resulting solution to form a stable solution, and removing a solvent used for preparing the liposome solution (preferably, removing the solvent used for preparing the liposome solution by rotary evaporation (preferably, in the rotary evaporation, a rotation speed is 80-120 rpm and a temperature is 40-60 °C; more preferably, the rotation speed is 80-100 rpm and the temperature is 45-60°C)).

12. The method according to claim 11, wherein the step of forming the hyaluronic acid liposome assembly further comprises: after the solvent used for preparing the liposome solution is removed, adding a buffer solution or water, uniformly mixing a resulting solution, and then passing the uniform mixed solution through an 800 nm filter membrane (preferably a polycarbonate filter membrane, a polytetrafluoroethylene filter membrane, a polypropylene filter membrane, or a polyvinylidene fluoride filter membrane) to form a primary hyaluronic acid liposome assembly; then adding a buffer solution or water to achieve a stable state and then passing the solution through a 450 nm filter membrane (preferably a polycarbonate filter membrane, a polytetrafluoroethylene filter membrane, a polypropylene filter membrane, or a polyvinylidene fluoride filter membrane); and repeating the above operation starting from passing the uniform mixed solution through an 800 nm filter membrane for 0-2 times to obtain the hyaluronic acid liposome assembly.

13. The method according to claim 12, wherein in the step of forming the hyaluronic acid liposome assembly, a mass percentage content of a primary hyaluronic acid liposome assembly in a solution obtained before the last passage through the 450 nm filter membrane is 45%-65%, and a mass percentage content of the hyaluronic acid liposome assembly in a solution obtained after the last passage through the 450 nm filter membrane is 25%-35%.

14. The method according to any one of claims 8 to 13, wherein the buffer solution is a phosphate buffer solution (preferably the phosphate buffer solution has a concentration of 1-10 mg/mL).

15. The method according to any one of claims 8 to 14, wherein in the step of adding the hyaluronic acid solution to the liposome solution under stirring condition to form the hyaluronic acid liposome assembly, the hyaluronic acid solution is added to the liposome solution at 20-40°C (preferably 25-30°C).

16. A hyaluronic acid liposome assembly prepared by the method according to any one of claims 4 to 15.

17. Use of the hyaluronic acid liposome assembly according to any one of claims 1 to 3 and 16 in the preparation of a transdermal absorption product (preferably a transdermal absorption drug or a transdermal absorption skin care product).
